# EUROPEAN PATENT APPLICATION

(11) **EP 1 977 739 A1**
(43) Date of publication of application: **08.10.2008**
(21) Application number: 07380089.8
(22) Date of filing: 03.04.2007
(51) Int. Cl.: A61K 9/51, A61K 31/737, A61K 31/722, A61K 31/738, A61K 31/715

(54) **Nanoparticulate composition of chitosan and chondroitin sulfate**

(71) Applicant: BIOIBERICA, S.A., 08029 Barcelona (ES)
(72) Inventor: Escaich Ferrer, Josep, 08021 Barcelona (ES); Alonso Fernandez, Ma José, 15782 Santiago de Compostella (ES); Vila Pena, Ana Isabel, 15782 Santiago de Compostella (ES); Ruhi Roura, Ramon, 08025 Barcelona (ES)
(74) Representative: Bernardo Noriega, Francisco

(57) **Abstract**

The invention relates to a composition comprising nanoparticles with a size of less than 1 micron for the release of chondroitin sulfate, wherein the nanoparticles comprise at least chitosan or a derivative thereof, and at least one chondroitin sulfite or a derivative thereof, and wherein the nanoparticles are crosslinked by means of a crosslinking agent. It also refers to a process for preparing said nanoparticulate composition.

## Description

### FIELD OF THE INVENTION

The invention relates to a composition comprising nanoparticles useful for the release of chondroitin sulfate. More specifically, it is aimed at compositions comprising nanoparticles of chitosan, chondroitin sulfate and optionally a polyoxyethylenated derivative, and which are ionically crosslinked, as well as processes for obtaining them.

### BACKGROUND OF THE INVENTION

The administration of biologically active molecules for therapeutic purposes presents numerous difficulties, both depending on the route of administration and the physicochemical and morphological characteristics of the molecules. It is known that the main drawbacks arise when administering unstable or large-sized active molecules. Access of macromolecules to the interior of the organism is limited by the low permeability of the biological barriers. Likewise, they are susceptible of being degraded due to the different defence mechanisms that both human and animal organisms have. Some drugs, amongst which are peptides, proteins and polysaccharides, are not effectively absorbed through mucosal surfaces due to the limited permeability of the epithelial barriers in the human and animal body.

One example of these drugs having little capacity to cross mucosal barriers and susceptible of being degraded in the organism is chondroitin sulfate.

Chondroitin sulfate is a sulfated glycosaminoglycan ususally found attached to proteins as part of a proteoglycan. It has been used for treating various pathological conditions of cartilaginous joints, such as osteoarthritic and inflammation processes, it can also perform a protective action on cartilaginous tissue and synovial fluid. Particularly important is its use for treating arthrosis, since it is regarded as being able to protect and reconstitute articular cartilage damaged by degenerative phenomena. Its use in the treatment of psoriasis has also been described (WO2005/014012).

To achieve the desired therapeutic effects, the product is commonly administered orally or parenterally, in its pure form or in the form of pharmaceutical compositions. In general, chondroitin sulfate is poorly soluble in water due to its polymeric nature, with a tendency to form lumps which hydrate extremely slowly. In therapy, said active principle is mostly administered by oral means with high doses, ranging from 400 to 1200 mg, for therapy periods lasting for months.

In the state of the art some pharmaceutical compositions containing chondroitin sulfate have hitherto been known for oral use, in particular in the form of tablets, capsules and granulated active principle contained in sachets for suspending in water prior to administration. A further pharmaceutical form employed is an oral gel, available is special sealed sachets to be opened at the time of consumption. Other composition described in the art refers to a chewable solid non-compressed pharmaceutical formulation for oral administration which tries to overcome the problems related to the size of the compositions mentioned above due to the high daily doses required for the treatment of the mentioned disorders (EP 1570842).

Although said pharmaceutical formulations have shown some therapeutic effect, it is known that the chondroitin sulfate provided to the body is substantially degraded in vivo. This degradation reduces the quantity of precursor molecules capable of being used for the reconstruction on the connective tissue and, consequently, the expected therapeutic benefit, in particular where these molecules are administered by the oral route. In order to enhance the therapeutic efficacy of chondroitin, a pharmaceutical composition and dietary supplements based on chitosan and chondroitin sulfate have been described for oral administration (US 6,599,888). Said compositions are in the form of tablets, capsules, powder for oral suspension, oral solutions, etc...

However, the different pharmaceutical compositions of the state of the art do not provide, after administration, an effective absorption of chondroitin sulfate through epithelial barriers, having also a little capacity to cross mucosal barriers. Thus it is necessary to develop administration systems which overcome said problems.

It has been demonstrated that the incorporation of macromolecules in nanometric-sized systems makes it easier for them to penetrate the epithelial barriers and protects them from being degraded. The design of nanoparticulate systems capable of interacting with said barriers is presented as a promising strategy in order to achieve the penetration of active ingredients through mucous membranes.

It is also known that the capacity of these systems to cross external barriers and access the interior of the organism both depends on their size and on their composition. Small-sized particles will increase the degree of transport with respect to those of larger size; nanoparticles, with diameter less than 1 µm, respond to these criteria. If they are prepared from polymers of natural and biocompatible origin, the possibilities increase of them being naturally transported through the organism's mucous membranes, by known transport mechanisms and without altering the epithelial physiology.

In this sense, chitosan has been used as natural and biocompatible polymer in the formulation of nanoparticulate systems (WO-A-01/32751, WO-A-99/47130, ES 2098188) due to the fact that it provides a positive charge to the nanoparticles which allows the absorption through a biological environment of anionic character and/or its adhesion to negatively charged biological membranes.

Despite the numerous publications aimed at developing compositions for the administration of chondroitin sulfate, there is a great need for providing a type of small size particulate system of easy production and with high yields, having a great capacity for association with chondroitin sulfate and which allows release thereof through mucosa with an improved permeability.

### BRIEF DESCRIPTION OF THE INVENTION

The inventors have found that a composition comprising nanoparticles that comprise chitosan and chondroitin sulfate, optionally in the presence of a polyoxyethylenated derivative, and obtained by means of an ionic gelling process in the presence of an agent causing reticulation, allows the effective association of chondroitin sulfate, as well as the subsequent release thereof in a suitable biological environment. The experimental data have shown that the presence of the crosslinking or reticulating agent is extremely important since it allows improving the nanoparticle production yield more than three times with respect to a system lacking it.

Surprisingly, these nanoparticles are stable in gastrointestinal fluids and present an excellent effectiveness and bioavailability, such as demonstrated by the permeability data obtained *in vitro.* These systems are therefore very adequate for oral administration, avoiding the degradation of the chondroitin sulfate. In fact, the in vitro permeability results may clearly predict that the absorption of chondroitin sulfate to the plasma levels will be significantly higher than when compared to the absorption of chondroitin administered in solution.

Furthermore, the nanoparticulate composition proposed in the invention for association and release of chondroitin sulfate have numerous advantages such as (1) the chondroitin sulfate incorporation process is simple and does not require the use of toxic ingredients; (2) their physicochemical properties, specifically their size and surface charge, can be modulated according to the ratio of formulation components and their molecular weight; (3) they have an extraordinary chondroitin sulfate association capacity; and (4) they release said active molecule.

Therefore, an object of the present invention consists in a composition comprising nanoparticles with a size of less than 1 micron for the release of chondroitin sulfate, wherein the nanoparticles comprise at least chitosan or a derivative thereof, and at least one chondroitin sulfate or derivative thereof, and wherein said nanoparticles are crosslinked by means of a crosslinking agent.

In a preferred embodiment of the invention the crosslinking agent is a polyphosphate salt, preferably sodium tripolyphosphate.

In another embodiment of the invention the nanoparticles comprise:
a) between 50% and 90% by weight of chitosan or a derivative thereof, with respect to the total weight of the nanoparticles, and
b) between 10% and 50% by weight of chondroitin sulfate or a derivative thereof, with respect to the total weight of the nanoparticles.

In another embodiment, the chitosan-chondroitin nanoparticles further comprise a polyoxyethylenated compound, preferably a polyoxyethylene or an ethylene oxide-propylene oxide copolymer.

In another embodiment of the invention the nanoparticles present a surface electric charge or Z potential which varies between 0 mV and +60 mV, preferably the electric charge varies between +1 and +50 mV.

In a particular embodiment, the composition of the invention is pharmaceutical composition. In a preferred embodiment, the pharmaceutical composition is for administration through mucosa. In another preferred embodiment, the pharmaceutical composition is for oral administration.

In another particular embodiment, the composition of the invention is a nutraceutical composition.

In another embodiment of the invention, the nanoparticles are in lyophilised form.

Another object of the invention consists in a process for preparing a composition for the release of chondroitin sulfate such as that defined above and comprising:
a) preparing an aqueous solution comprising chitosan or a derivative thereof;
b) preparing an aqueous solution comprising chondroitin sulfate or a derivative thereof and the crosslinking agent; and
c) mixing, with stirring, the solutions of steps a) and b), such that the chitosan-chondroitin sulfate nanoparticles are obtained by means of ionic gelling.

In an embodiment of the invention, this process further comprises an additional step after step c) wherein the nanoparticles are lyophilised.

Finally, another object of the invention refers to the use of a composition as defined above for the elaboration of a medicament for the treatment and/or prevention of pathological conditions of cartilaginous joints selected from osteoarthrotic and inflammation processes; articular cartilage damaged by degenerative phenomena and arthrosis. It is also possible its use for the elaboration of a medicament for the treatment and/or prevention of psoriasis.

### DETAILED DESCRIPTION OF THE INVENTION

The composition of the present invention comprises nanoparticles whose structure is a reticulate of chitosan and chondroitin sulfate. The structure is held together by electronic interactions between the chitosan (with positive charge) and chondroitin sulfate (with negative charge), not being substantially any covalent bonding between them.

By the term "nanoparticle" it is understood a structure formed by the electrostatic interaction between the chitosan and the chondroitin sulfate and from the ionotropic gelification of the conjugate obtained by means of the addition of a reticulating agent. The electrostatic interaction resulting between both components of the nanoparticles and the subsequent reticulating generates characteristic physical entities, which are independent and observable, whose average size is less than 1 µm, i.e. an average size between 1 and 999 nm.

By the term "average size" it is understood the average diameter of the nanoparticle population, which comprises the polymeric reticulated structure, which moves together in an aqueous medium. The average size of these systems can be measured using standard procedures known by a person skilled in the art, and which are described, for example, in the experimental part below.

The nanoparticles comprised in the composition of the invention have an average particle size of less than 1 µm, i.e. they have an average size between 1 and 999 nm, preferably between 50 and 800 nm, more preferably between 100 and 700 nm, even more preferably between 100 and 600 nm. The average particle size is mainly influenced by the molecular weight of chitosan, by the degree of deacetylation of chitosan, by the proportion of chitosan with respect to the polyoxyethylenated derivative if present, and also by the particle formation conditions (chitosan concentration, crosslinking agent concentration and ratio between them). In general, the presence of the polyoxyethylenated derivative causes an increase in the mean particle size with respect to systems formed by chitosan without said derivative.

The nanoparticles may have a surface electric charge (measured by zeta potential) which varies depending on the proportion of the chitosan and chondroitin sulfate in the nanoparticles. The contribution to the positive charge is attributed to the amine groups of the chitosan, while the contribution to the negative charge is attributed to the sulfate groups of chondroitin sulfate. Depending on the chitosan/ chondroitin sulfate proportion and particularly on the degree of deacetylation and on the presence or absence of inorganic salts, the charge magnitude may vary between 0 mV and +60 mV, preferably between +1 and +50 mV.

Frequently, it is of interest that the surface charge is positive in order to improve the interaction between the nanoparticles and biological surfaces, particularly mucous surfaces, which are negatively charged. This way, the biologically active molecule will favourably act on the target tissues. However, in some instances, a neutral charge may be more suitable in order to ensure the stability of the nanoparticles following parenteral administration.

### Chitosan

Chitosan is a natural polymer which has an aminopolysaccharide structure and a cationic character. It comprises the repetition of monomer units of formula (I): 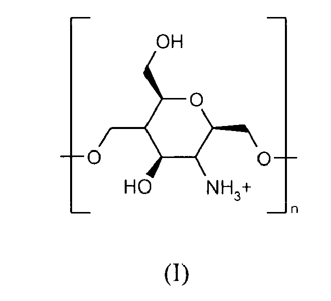 where n is an integer and represents the degree of polymerisation, i.e. the number of monomer units in the chitosan chain.

In addition to these monomer units, chitosan generally contains a proportion of monomer units in which the amino group is acetylated. In fact, chitosan is obtained by deacetylation of chitin (100% acetylated). Said degree of deacetylation is generally within a range comprised between 30 and 95, preferably between 55 and 90, which indicates that between 10 and 45% of the amino groups are acetylated.

The chitosan used to obtain the nanoparticles of the present invention has a molecular weight comprised between 2 and 2000 kDa, preferably between 2 and 500 kDa, more preferably between 10 and 200 kDa.

As an alternative to chitosan, a derivative thereof can also be used, understanding as such a chitosan wherein one or more hydroxyl groups and/or one or more amine groups have been modified, with the aim of increasing the solubility of the chitosan or increasing the adhesive nature thereof. These derivatives include, among others, acetytated, alkylated or sulfonated chitosans, thiolated derivatives, as is described in Roberts, Chitin Chemistry, Macmillan, 1992, 166. Preferably, when a derivative is used it is selected from O-alkyl ethers, O-acyl esters, trimethyl chitosan, chitosans modified with polyethylene glycol, etc. Other possible derivatives are salts, such as citrate, nitrate, lactate, phosphate, glutai-nate, etc. In any case, a person skilled in the art knows how to identify the modifications which can be made on the chitosan without affecting the stability and commercial feasibility of the end formulation.

### Condroitin sulfate

Chondroitin sulfate is a sulfated glycosaminoglycan (GAG) composed of a chain of alternating sugars. It is usually found attached to proteins as part of a proteoglycan. The molecular weight of chondroitin sulfate ranges from 15 to 150 basic units of N-acetylgalactosamine and glucuronic acid. It is represented by the following structure: 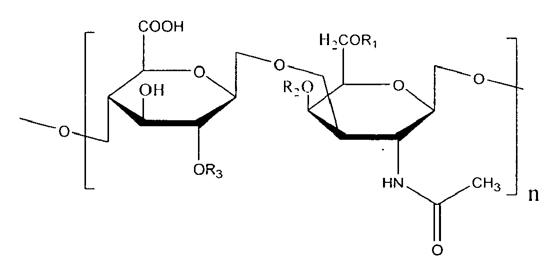 where n is an integer and represents the degree of polymerisation, i.e. the number of disaccharide units in the chondroitin sulfate chain and wherein R₁, R₂ and R₃ are independently hydrogen or a SO₃H group. Each monosaccharide may be left unsulfated, sulfated once, or sulfated twice. Sulfation is mediated by specific sulfotransferases.

The term 'chondroitin sulfate' includes the following substances:
- chondroitin sulfate A which is predominantly sulfated at carbon 4 of the N-acetylgalactosamine (GaINAc) sugar and is also known as chondroitin-4-sulfate (R₁=H, R₂=SO₃H and R₃=H)
- chondroitin sulfate B is referred to as dermatan sulphate. This substance is made up of linear repeating units containing N-acetylgalactosamine and either L-iduronic acid or glucuronic acid, and each disaccharide may be sulfated once or sulfated twice.
- chondroitin sulfate C which is predominantly sulfated at carbon 6 of the GalNAc sugar and is also known as chondroitin-6-sulfate (R₁=SO₃H, R₂=H and R₃=H);
- chondroitin sulfate D which is predominantly sulfated at carbon 2 of the glucuronic acid and at carbon 6 of the GaINAc sugar and is also known as chondroitin-2,6-sulfate (R₁=SO₃H, R₂=H and R₃= SO₃H);
- chondroitin sulfate E which is predominantly sulfated at carbons 4 and 6 of the GalNAc sugar and is also known as chondroitin-4,6-sulfate (R₁= SO₃H, R₂=SO₃H and R₃= H).

In the present description, the term chondroitin sulfate encompasses all these substances or mixtures thereof.

Although the name "chondroitin sulfate" suggests a salt with a sulfate as counter-anion, this is not the case, since the sulfate is covalently attached to the sugar. Rather, since the molecule has multiple negative charges at physiological pH, a cation may be present in salts of chondroitin sulfate. In fact, commercial preparations of chondroitin sulfate are typically in the form of its sodium salt. Therefore, the term 'chondroitin sulfate' also includes organic and inorganic salts thereof. Generally, such salts are, for example, prepared by reacting the base form of this compound with a stoichiometric amount of the appropriate acid in water or in an organic solvent or in a mixture of the two. Generally, nonaqueous media like ether, ethyl acetate, ethanol, isopropanol or acetonitrile are preferred. Examples of inorganic salts include, for example, sodium, potassium, calcium, ammonium, magnesium, aluminium and lithium salts, and organic salts include, for example, ethylenediamine, ethanolamine, *N,N-*dialkylenethanolamine, triethanolamine, glucamine and basic aminoacids salts.

Chondroitin's functions largely depend on the properties of the overall proteoglycan of which it is a part. These functions can be broadly divided into structural and regulatory roles. However, this division is not absolute and some proteoglycans have both structural and regulatory roles.

Regarding its structural role, chondroitin sulfate is a major component of extracellular matrix, and is important in maintaining the structural integrity of the tissue. As part of aggrecan, chondroitin sulfate is a major component of cartilage. The tightly packed and highly charged sulfate groups of chondroitin sulfate generate electrostatic repulsion that provides much of the resistance of cartilage to compression.

Amongst the known applications of chondroitin sulfate or their derivatives are the prevention and treatment of various pathological conditions of cartilaginous joints, such as osteoarthritic and inflammation processes, and can also perform a protective action on cartilaginous tissue and synovial fluid. Particularly important is its use for treating arthrosis, since it is regarded as being able to protect and reconstitute articular cartilage damaged by degenerative phenomena.

The composition of the invention, comprising nanoparticles for the administration of chondroitin sulfate or derivatives thereof, preferably has a chitosan or a chitosan derivative content comprised between 50 and 99% by weight with respect to the total weight of the nanoparticles, preferably between 50% and 90% by weight. On the other hand, the chondroitin sulfate content in the system is preferably comprised between 10 and 50% by weight with respect to the total weight of the nanoparticles, preferably between 25% and 40% by weight.

The chitosan-chondroitin sulfate nanoparticle composition of the invention is characterised in that it has been formed by means of a joint precipitation process of chitosan and chondroitin sulfate in the form of polymeric nanoclusters caused by the addition of a crosslinking agent. The use of organic solvents or extreme pH conditions or toxic auxiliary substances is not required. The association of chondroitin sulfate to the nanoparticles occurs according to an ionic interaction mechanism. Chondroitin sulfate has numerous negative groups in its structure, sulfate and carboxylate groups, which justifies a high ionic affinity for the positive amino groups of chitosan, favouring nanoparticle appearance. The presence of the crosslinking agent allows crosslinking of the chitosan- chondroitin sulfate system so that a compact lattice is formed between which the chondroitin sulfate is inserted which may subsequently be released. Furthermore, the crosslinking agent provides the nanoparticles with their size, potential and structural characteristics which make them suitable as an administration system for said active molecule.

The crosslinking agent is preferably an anionic salt allowing reticulation of the chitosan- chondroitin sulfate conjugate by means of ionic gelling, causing the spontaneous formation of nanoparticles. Preferably a polyphosphate salt is used, sodium tripolyphosphate (TPP) being especially preferred. In a particular embodiment of the aforementioned composition, the crosslinking agent/chitosan weight ratio is comprised between 0.001/1 and 0.50/1, the 0.01/1 and 0.40/1 weight ratio being preferred, which provides formulations with a relatively low polydispersity. However, the use of larger or smaller crosslinking agent/chitosan weight ratios is also possible.

In a particular embodiment of the composition of the invention, the nanoparticles may include a polyoxyethylenated compound. A polyoxyethylenated compound is understood as a synthetic hydrophilic polymer of non-ionic character having units of ethylene oxide in its structure, the use of a polyoxyethylene or an ethylene oxide-propylene oxide (PEO-PPO) copolymer, commonly called poloxamers, being preferred. These polymers are commercially available with different molecular weights, however, in the present invention the use of polyoxyethylenated derivatives having molecular weights comprised between 2000 and 10000 is preferred. In a preferred embodiment, the polyoxyethylenated derivative is a triblock copolymer (PEO-PPO-PEO), such as for example that commercially called Poloxamer 188.

The proportion of chitosan with respect to the polyoxyethylenated compound which is incorporated to the particle formation medium may be very variable, ranging between 50:0 and 1:100, preferably between 50:0 and 1:20. The presence of the polyoxyethylenated compound may have as an effect slightly increasing the size of the nanoparticles and it aids in stabilising the colloidal system. If it is added subsequently to nanoparticle formation it forms a coating which significantly increases the size thereof. Although the presence of the polyoxyethylenated derivative is not necessary for obtaining the nanoparticles, it allows modifying the physicochemical characteristics of said nanoparticles (size and zeta potential), and above all, stabilising the system in gastrointestinal fluids.

The preparation and formation of chitosan nanoparticles and an active ingredient, by means of a crosslinking agent in the presence or absence of a polyoxyethylenated compound, is described in WO98/04244, which is herein considered incorporated in its entirety by reference.

In a particular embodiment, the composition of the invention is a pharmaceutical composition. Although said composition is mainly for administration through mucosae, the pharmaceutical composition of the invention may be administered by oral, buccal or sublingual, transdermal, ocular, nasal, vaginal or parenteral route. In the case of the non-parenteral routes the contact of the nanoparticles with the skin or mucosae can be improved by providing the particles with an important positive charge, which will favour their interaction with said negatively charged surfaces.

In a preferred embodiment, the pharmaceutical composition is administered by mucosal route. The positive charge of chitosan provides a better absorption of chondroitin sulfate on the mucosal surface through its interaction with the mucosa and the surfaces of the epithelial cells which are negatively charged.

In another preferred embodiment, the pharmaceutical composition is administered by oral route. In this case, the nanoparticles have the additional advantage that they are stable in gastrointestinal fluids, so they may reach and remain without degradation on the intestinal epithelial tissue so as to release the chondroitin sulfate.

In another particular embodiment, the composition of the invention is a nutraceutical composition. Said nutraceutical composition can be a food product, a dietetic supplement or a nutritional supplement. Nutraceutical compositions can include milk, dairy products, yoghurts, fermented milks, fruit and vegetable juices, biscuits, cakes, infant food and dehydrated food. The addition of the nanoparticles of the invention to the nutraceutical composition is effected by mixture and homogenisation according to the technological procedure of each product.

Other components such as vitamins can be added to the nutraceutical composition. Thus, vitamins A, B6, B12, C, D, E or folic acid or a mixture of one or more thereof can be added to the nutraceutical composition according to the present invention. The addition of these compounds can be effected either previously or after the heat treatment according to the specific technological procedure of each nutritional product.

Another embodiment of the present invention refers to a process for preparing chitosan-chondroitin sulfate nanoparticles such as those previously defined, comprising:
a) preparing an aqueous solution of chitosan or a derivative thereof;
b) preparing an aqueous solution of chondroitin sulfate and of the crosslinking agent; and
c) mixing, with stirring, of the solutions of steps a) and b), such that the chitosan- chondroitin sulfate nanoparticles are spontaneously obtained by means of ionic gelling and subsequent precipitation.

In a particular embodiment of the aforementioned process, the resulting crosslinking agent/chitosan weight ratio is comprised between 0.001/1 and 0.50/1, the 0.01/1 and 0.40/1 weight ratio being preferred, which provides formulations with a relatively low polydispersity. However, the use of larger or smaller crosslinking agent/chitosan ratios is also possible.

In another embodiment of the process, it further comprises incorporating the polyoxyethylenated compound to the aforementioned aqueous solution of chitosan.

In another embodiment of the process for obtaining the nanoparticles of the invention, inorganic salts may be used, such as for example sodium chloride or sodium acetate (from acetic acid and sodium hydroxide), which allows increasing the nanoparticle production yield. They may be added to the chitosan or chitosan derivative solution. However, it has been observed that incorporating said salts modifies the interaction between chitosan and chondroitin sulfate, causing a slight decrease in the amount of chondroitin sulfate associated to the nanoparticulate system, as well as neutralising the nanoparticle charge because the negative ions of the medium are absorbed into the positive surface of the chitosan. Taking this into account, a person skilled in the art may use said salts, according to the desired final characteristics, in the case of low yields, and in amounts leading to a suitable yield but without negatively affecting the degree of association or the charge if this is desired to be sufficiently high as to improve interactions with the mucosa.

In yet another embodiment of the process, if it is desired that the chitosan-chondroitin sulfate nanoparticles are to be coated by the polyoxyethylenated compound, the latter is incorporated after nanoparticle formation.

These processes for preparing the nanoparticles of the invention allow an association of chondroitin sulfate to the nanoparticles exceeding 80%. Slight variations in said association can be observed depending on the molecular weight of the chitosan, its degree of deacetylation and the presence of the polyoxyethylenated derivative or inorganic salts.

Once the nanoparticles are formed, they may be isolated by means of ultrafiltration or centrifugation in a glycerol or glucose bed, or in a trehalose solution, discarding the supernatant, with the object of separating the chondroitin sulfate molecules which are not associated to the nanoparticles. The nanoparticles can subsequently be resuspended in water or buffer for their use in suspension.

The process for preparing the chitosan-chondroitin sulfate nanoparticles can further comprise an additional step in which said nanoparticles are subjected to lyophilisation. From a pharmaceutical point of view it is important to be able to have the nanoparticles available in lyophilised form since this improves their stability during storage. The chitosan-chondroitin sulfate nanoparticles (in which chitosan may be physically mixed with a polyoxyethylenated derivative) may be lyophilised in the presence of a cryoprotectant, such as glucose, sucrose or trehalose, at a 5% concentration. In fact, the nanoparticles of the invention have the additional advantage that the particle size before and after lyophilisation is not significantly modified. That is, the nanoparticles can be lyophilised and resuspended without an alteration in the characteristics thereof.

Finally, another object of the invention refers to the use of a composition as defined above for the elaboration of a medicament for the treatment and/or prevention of pathological conditions of cartilaginous joints selected from osteoarthrotic and inflammation processes; articular cartilage damaged by degenerative phenomena and arthrosis. It is also possible its use for the elaboration of a medicament for the treatment and/or prevention psoriasis.

Some illustrative examples are described below which show the features and advantages of the invention; they should not be interpreted as limiting the object of the invention.

### EXAMPLES

As a common process to all the examples detailed below, the nanoparticles are characterised from the point of view of size, zeta potential (or surface charge), association effectiveness (percent of chondroitin sulfate that has been associated to the nanoparticles) and production yield (percent of materials forming the nanoparticles).

The *Size Distribution* has been performed by means of photon correlation spectroscopy (PCS; Zeta Sizer, Nano series, Nano-ZS, Malvern Instruments, UK), giving mean size and nanoparticle population dispersion (polydispersity index) values.

The *Zeta Potential* has been measured by Laser Doppler Anemometry (LDA; Zeta Sizer, Nano series, Nano-ZS, Malvern Instruments, UK). The samples were diluted in Milli-Q water so as to determine electrophoretic mobility.

*Quantification or the amount of associated chondroitin sulfate* has been determined sulfates levels by capillary electrophoresis (or ionic HPLC) after calcinations (and treatment with nitric and hydrochloric acids) of the samples corresponding to the original formulations and to those in the basal compartments.

The *Production yield* was quantified by means of nanoparticle centrifugation at 16000 xg for 40 minutes, subsequent removal of the supernatant (where the components remaining in solution will be) and finally, weighing the dry residue.

The chitosan (Protasan UP Cl 113) used in the examples comes from NovaMatrix-FMC Biopolymer, the chondroitin sulfate (sodium salt) from Bioibérica S.A., and the remaining products used, such as sodium tripolyphosphate, are from Sigma Aldrich.

### Example 1

### Preparation and characterisation of chitosan-chondroitin sulfate nanoparticles

A 3 mL aqueous solution of chitosan (CS) (1 mg/mL) is subjected to magnetic stirring while a 0.6 mL aqueous solution of chondroitin sulfate (2 mg/mL) and pentasodium tripolyphosphate (TPP, 0.75 mg/mL) is added thereto, forming thus spontaneously the nanoparticles. The final weight ratio chitosan:chondroitin sulfate:pentasodium tripolyphosphate is respectively 1:0.4:15. Table I shows the results obtained for the different parameters (size, Zeta potential and association effectiveness) of the nanoparticles.

**Table I: Physico-chemical characterization of nanoparticles and chondroitin association effectiveness.**

| size (nm) | Zeta potential (mV) | association effectiveness (%) |
|---|---|---|
| 271±33 | +36.51±7.16 | 81±2 |

As can be seen from the obtained results, the nanoparticles are characterized for having an average size less than 1 micron and a positive Zeta potential due to the presence of chitosan. The high association effectiveness of chondroitin sulfate should be highlighted, with about 80% of chondroitin associated to the reticulated system.

### Example 2

### Influence of the presence of crosslinking agent in the preparation of nanoparticles of chitosan and chondroitin sulfate.

Nanoparticles of chitosan and chondroitin sulfate were prepared according to the procedure described in example 1, to show the effect of the presence or absence of pentasodium tripolyphosphate (TPP) as crosslinking agent. The nanoparticle production yields were measured in both cases and the results are shown in Table II.

**Table II**

| Formulation | Production yield (%) |
|---|---|
| Complexes (without TPP) | 20.8±1.2 |
| Nanoparticles (with TPP) | 65.0±3.8 |

The results show that the crosslinking agent is necessary in the preparation of chitosan-chondroitin sulfate nanoparticles. When no TPP is added to the formulation, only complexes of chitosan and chondroitin sulfate are obtained due to the interaction between the positive charge groups of the chitosan and the negative charge groups of the chondroitin sulfate. But these complexes have a structure similar to hydrogels, which are not convenient as pharmaceutical form. However, when TPP is added, nanoparticles are formed with a more compact and dense structure favouring the interaction between both polysaccharide components and improving the nanoparticles production yield. Additionally, the nanoparticles are good vehicles for the administration of biologically active molecules.

### Example 3

### Influence in the nanoparticle average size when larger quantity of nanoparticles are prepared.

When nanoparticles are obtained according to the procedure described in example but increasing the total volume from 3.6 mL to 110 mL, it has been observed that the average size is maintained (see Table III). Only a little increase in the size is observed when increasing the concentration of chitosan.

**Table III: Average size of chitosan-chondroitin sulfate nanoparticles obtained in a 110 mL solution.**

| chitosan concentration (mg/mL) | average size (nm) |
|---|---|
| 1 | 242 |
| 1.25 | 321 |
| 1.5 | 398 |

These results show that the nanoparticles of the invention can be produced efficiently on an industrial scale.

### Example 4

### In vitro effectiveness of chondroitin sulfate in solution and associated to the chitosan nanoparticles. Permeability coefficients determination.

Nanoparticles of chitosan and chondroitin sulfate were prepared according to the procedure described in example 1, with the exception that 0.4 mg/mL of chondroitin sulfate was added to the aqueous solution containing TPP.

In addition, an aqueous solution of 0.4 mg/mL of chondrotin sulfate was prepared for the purpose of comparative analysis.

With the aim of predicting and comparing the bioavailability of chondroitin sulfate in solution and associated to the nanoparticulate system, an in vitro permeability analysis was carried out using a CacoGlobet cellular system (co-culture of enterocytic cells Caco-2 with mucosecreting cells HT29-M6). This cellular system simulates the intestinal epithelium because it displays synthesis and secretion of mucous, a barrier that limits in most cases the absorption of the therapeutic molecules by the epithelial membrane.

Three independent experiments were performed with an incubation time of 2 hours and in a sextuplicate form for both assayed formulations.

The analytical method followed in this study was the determination of sulfates by capillary electrophoresis (or ionic HPLC) after calcinations (and treatment with nitric and hydrochloric acids) of the samples corresponding to the original formulations and to those in the basal compartments.

The data obtained for the analysis of barrier integrity (TEER and "Lucifer Yellow") were very similar in the three experiments and therefore, there were no significant differences between them.

The statistical comparison between both formulations, as shown in Table IV, was achieved using the non-parametric test of Mann-Whitney. The results pointed out that significant differences exist (p<0.05) between the permeability coefficients of chondroitin sulfate in solution and that associated to chitosan nanoparticles.

**Tabla IV: Resulting permeability coefficients**

| Pₐₚₚ (cm/s) | | | | | | | |
|---|---|---|---|---|---|---|---|
| | solution of chondroitin sulfate | | | chitosan nanoparticles | | | |
| | Exp.1 | Exp.2 | Exp.3 | Exp.1 | | Exp.2 | Exp.3 |
| | 1,469E-05 | 1,406E-05 | 8,508E-06 | 1,0501,-05 | | 2,472E-05 | 3,575E-05 |
| | 1,497E-06 | 1,234E-05 | 2,933E-06 | 3,960E-06 | | 3,900E-05 | 1,481E-05 |
| | 3,578E-06 | 1,835E-05 | 4,606E-07 | 1.781E-05 | | 2,613E-05 | 4,647E-05 |
| | 1,051E-05 | | 3,854E-06 | 2.426E-05 | | 4,471E-05 | 3,611E-05 |
| | 1,110E-05 | | 1,699E-05 | 1,353E-05 | | 3,628E-05 | 2,663E-05 |
| | 1,346E-05 | | | 2,136E-05 | | 2,200E-05 | 3,695E-05 |
| | | | | | | | |
| Average | 9.452E-06 | | | | 2.672E-5 | | |
| Desvest | 5.989E-6 | | | | 1.205 E-5 | | |

Taking into account the following premises, the in vivo oral behaviour may be predicted for each tested formulation in the cellular cultures):
Pₐₚₚ < 10⁻⁶ cm/s: low oral absorption in humans
10⁻⁶ < Pₐₚₚ < 10⁻⁵ cm/s: moderate oral absorption
Pₐₚₚ> 10⁻⁵ cm/s: high oral absorption

Therefore, the in vitro permeability results clearly predict that the oral absorption of chondroitin sulfate to the plasma levels will be significantly higher when administered in the nanoparticles of the invention than when compared to the absorption of chondroitin administered in solution.

## Claims

1. A composition comprising nanoparticles with a size of less than 1 micron for the release of chondroitin sulfate, wherein the nanoparticles comprise at least chitosan or a derivative thereof, and at least one chondroitin sulfate or a derivative thereof, and wherein the nanoparticles are crosslinked by means of a crosslinking agent.

2. A composition according to claim 1, wherein the crosslinking agent is a polyphosphate salt, preferably sodium tripolyphosphate.

3. A composition according to claim 1 or 2, wherein the nanoparticles comprise:
a) between 50% and 90% by weight of chitosan or a derivative thereof, with respect to the total weight of the nanoparticles, and
b) between 10% and 50% by weight of chondroitin sulfate or a derivative thereof, with respect to the total weight of the nanoparticles.

4. A composition according to any of claims 1 to 3, wherein the nanoparticles further comprise a polyoxyethylenated compound.

5. A composition according to claim 4, wherein the polyoxyethylenated compound is a polyoxyethylene or an ethylene oxide-propylene oxide polymer.

6. A composition according to claim 4, wherein the proportion of chitosan or a derivative thereof with respect to the starting amount of the polyoxyethylenated compound is comprised between 50:1 and 1:50 by weight, preferably between 50:1 and 1:20.

7. A composition according to any of claims 1 to 6, wherein the chitosan or a derivative thereof has a molecular weight comprised between 2 and 2000 kDa, preferably between 2 and 500 kDa, more preferably between 10 and 200 kDa.

8. A composition according to any of claims 1 to 7, wherein the chitosan or a derivative thereof has a degree of deacetylation comprised between 30% and 95%, preferably between 55% and 90%.

9. A composition according to any of claims 1 to 8, wherein the crosslinking agent and chitosan weight ratio is comprised between 0.001:1 and 0.50:1, preferably between 0.01:1 and 0.40:1.

10. A composition according to any of claims 1 to 9, wherein the electric charge (Z potential) of the nanoparticles is comprised between 0 mV and +60 mV, preferably between +1 and +50 mV.

11. A composition according to any of claims 1 to 10 which is a pharmaceutical composition.

12. A pharmaceutical composition according to claim 11 for administration through mucosa.

13. A pharmaceutical composition according to claim 11 for oral administration.

14. A composition according to any of claims 1 to 10 which is a nutraceutical composition.

15. A nutraceutical composition according to claim 14 which is a food product, a dietetic supplement or a nutritional supplement.

16. A nutraceutical composition according to claims 14 or 15 which is milk, dairy products, yoghurts, fermented milks, fruit and vegetable juices, biscuits, cakes, infant food and dehydrated food.

17. A composition according to any of claims 1 to 16 wherein the nanoparticles are in lyophilized form.

18. A process for preparing a composition for the administration of chondroitin sulfate or a derivative thereof as defined in any of claims 1 to 10 comprising:
a) preparing an aqueous solution comprising chitosan or a derivative thereof;
b) preparing an aqueous solution comprising chondroitin sulfate or a derivative thereof and the crosslinking agent; and
c) mixing, with stirring, the solutions of steps a) and b), such that the chitosan-chondroitin sulfate nanoparticles are spontaneously obtained by means of ionic gelling.

19. A process according to claim 18, wherein the aqueous solution of step a) further comprises a polyoxyethylenated compound.

20. A process according to claims 18 or 19, wherein the aqueous solution of step a) further comprises inorganic salts, preferably sodium chloride or sodium acetate.

21. A process according to claims 18 to 20, wherein the crosslinking agent is a tripolyphosphate, preferably sodium tripolyphosphate.

22. A process according to claims 18 to 21 which further comprises an additional step after step c) in which the nanoparticles are lyophilised.

23. A process according to claim 22 wherein the nanoparticles are lyophilised in the presence of a cryoprotectant selected from glucose, sucrose and ti-elialose.

24. Use of a composition as defined in any of claims 1 to 13 for the elaboration of a medicament for the treatment and/or prevention of pathological conditions of cartilaginous joints selected from osteoarthrotic and inflammation processes; articular cartilage damaged by degenerative phenomena and arthrosis.

25. Use of a composition as defined in any of claims 1 to 13 for the elaboration of a medicament for the treatment and/or prevention of psoriasis.
